# EUROPEAN PATENT APPLICATION

(11) **EP 2 359 788 A1**
(43) Date of publication of application: **24.08.2011**
(21) Application number: 09831629.2
(22) Date of filing: 19.11.2009
(51) Int. Cl.: A61F 13/49, A61F 13/15, A61F 13/494, A61F 13/53

(54) **WEARING ARTICLE**

(30) Priority: 11.12.2008 JP 2008316246
(71) Applicant: Unicharm Corporation, Ehime 799-0111 (JP)
(72) Inventor: SUZUKI, Maiko, Kanonji-shi Kagawa 769-1602 (JP); MIZUTANI, Katsumi, Kanonji-shi Kagawa 769-1602 (JP)
(74) Representative: Stark, Gordon Drummond
(86) International application number: PCT/JP2009/006217
(87) International publication number: WO 2010/067521

(57) **Abstract**

The present invention provides a wearing article improved so as to prevent bodily fluids from staying in the proximal side edges of the cuffs and thereby to restrict development of skin troubles such as diaper rashes due to such staying bodily fluids.

A chassis 20 of a diaper 10 includes an inner sheet 21 lying on a side facing the wearer's body and an outer sheet 22 lying on a side facing away from the wearer's body wherein a liquid-absorbent structure 40 is sandwiched between these inner and outer sheet 21, 22. The liquid-absorbent structure 40 is formed with highly absorbent regions having a higher bodily fluid absorption capacity than that of a remaining region. The highly absorbent regions include front regions 43 lying on the side of a front end 14 and rear regions 44 lying on the side of a rear end 15. The highly absorbent regions are formed by increasing a thickness of a liquid-absorbent core 41. Leakage-barrier cuffs 30 are formed on the side of the inner sheet 21 facing the wearer's body. Proximal side edges 31 of the leakage-barrier cuffs 30 are bonded to the inner sheet 21 by bonding means 34 which partially overlap the front and rear regions 43, 44 of the liquid-absorbent structure 40.

## Description

### Technical Field

The present invention relates to wearing articles and particularly to wearing articles such as disposable diapers, toilet-training pants or incontinent briefs.

### Background Art

Conventionally, a disposable clothing material as one typical type of wearing articles is known, for example, from JP 2008-534230 A (Patent Literature 1). The disposable clothing material disclosed in Patent Literature 1 comprises the topsheet, the backsheet, the liquid-absorbent assembly sandwiched between these top- and backsheets and the leakage-barrier cuffs formed on the side of the topsheet facing the wearer's body. Each of the leakage-barrier cuffs has the proximal side edge bonded to the portion of the backsheet extending outward beyond the topsheet and the distal side edge adapted to be spaced from the liquid-absorbent assembly by the intermediary of the topsheet. The proximal side edges are located outside the opposite side edges of the liquid-absorbent assembly. When the disposable clothing material is put on the wearer's body, the distal side edges of the leakage-barrier cuffs are spaced upward from the liquid-absorbent assembly so as to stand toward the wearer's body. The standing leakage-barrier cuffs serve to prevent bodily fluids such as urine from leaking sideways.

### Citation List

### Patent Literature

Patent Literature 1: JP 2008-534230 A

### Summary of Invention

### Technical Problem

Since The proximal side edges are located outside the opposite side edges of the liquid-absorbent assembly, regions in which the liquid-absorbent assembly is not present are left inside the proximal side edges of leakage-barrier cuffs. Bodily fluids prevented by the proximal side edges of the leakage-barrier cuffs flow along the proximal side edges of the leakage-barrier cuffs toward the liquid-absorbent assembly. However, the liquid-absorbent assembly is not present in the proximal side edges of the leakage-barrier cuffs and there is a possibility that bodily fluids might stay in the proximal side edges. In consequence, it is apprehended that such bodily fluids staying in this manner might cause skin troubles such as diaper rashes.

An object of the present invention is to provide a wearing article improved so as to prevent bodily fluids from staying in the proximal side edges of the cuffs and thereby to restrict development of skin troubles such as diaper rashes caused by such staying bodily fluids.

### Solution to Problem

According to the present invention, there is provided a wearing article comprising a chassis having a longitudinal direction and a transverse direction, and comprising a side facing the wearer's body, a side facing away from the wearer's body, a front waist region, a rear waist region and a crotch region extending between the front and rear waist regions wherein these regions are continuous in the longitudinal direction, a liquid-absorbent structure located at least in the crotch region, and a pair of leakage-barrier cuffs lying on the side of the chassis facing the wearer's body and spaced from each other in the transverse direction.

The present invention is characterized in that the leakage-barrier cuffs respectively include proximal side edges extending in the longitudinal direction and secured to the chassis, free side edges adapted to be spaced upward from the chassis and cuff elastics attached under tension and in a contractible manner in the longitudinal direction to the free side edges; the liquid-absorbent structure includes highly absorbent regions having a higher bodily fluid absorption capacity than that in a remaining region; and the proximal side edges overlapped on the highly absorbent regions.

According to one embodiment of the present invention, the liquid-absorbent structure comprises liquid-absorbent core and a wrapping sheet used to wrap the liquid-absorbent core; and the highly absorbent regions are partially formed by increasing a thickness of the liquid-absorbent core so that the liquid-absorbent structure may have a thicker dimension in a thickness direction thereof than that of the remaining region.

According to another embodiment of the present invention, the liquid-absorbent structure comprises a liquid-absorbent core and a wrapping sheet used to wrap the liquid-absorbent core; and the highly absorbent regions are partially formed by setting a higher basis mass of said liquid-absorbent core than that of a remaining region.

According to still another embodiment of the present invention, the wearing article having fold lines extending in the transverse direction and along which the wearing article is folded; and the fold lines at least partially overlap the highly absorbent regions.

According to yet another embodiment of the present invention, the highly absorbent regions comprise front and rear regions spaced one from another in the longitudinal direction and the front and rear regions are formed continuously from the crotch region toward the front and rear waist regions.

According to further another embodiment of the present invention, the highly absorbent regions are formed to be spaced one from another in the transverse direction.

According to still further another embodiment of the present invention, the wearing article further includes a liquid-absorbent pad adapted to be placed on the side facing the wearer's body of the chassis between a pair of the leakage-barrier cuffs.

### Advantageous Effects of Invention

The liquid-absorbent structure adapted to absorb bodily fluids includes the highly absorbent regions overlapping the proximal side edges of the leakage-barrier cuffs so that the bodily fluids flowing along the proximal side edges can be quickly absorbed and contained by the liquid-absorbent structure. In other words, bodily fluids should not stay on the chassis in the vicinity of the proximal side edges. In consequence, skin troubles such as diaper rashes due to bodily fluids staying on the chassis can be restricted.

### Brief Description of Drawings

[FIG. 1] A perspective view of a sterically-assembled diaper.
[FIG. 2] A plan view corresponding to the diaper of Fig. 1 having been flatly developed.
[FIG. 3] A schematic sectional view taken along line III-III in Fig. 2.
[FIG. 4] A schematic sectional view taken along line IV-IV in Fig. 2.

### Description of Embodiments

Details of a wearing article according to the present invention will be more fully understood from the description of a disposable diaper as one typical example thereof with reference to the accompanying drawings.

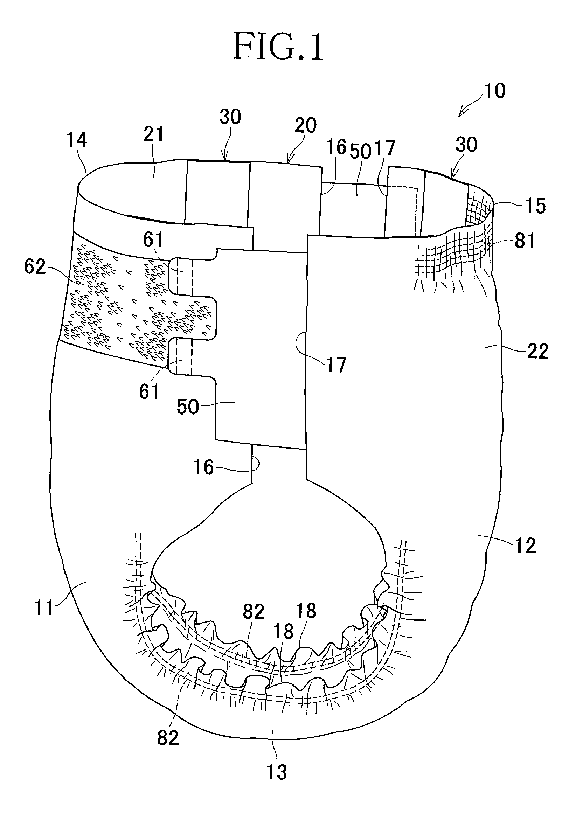

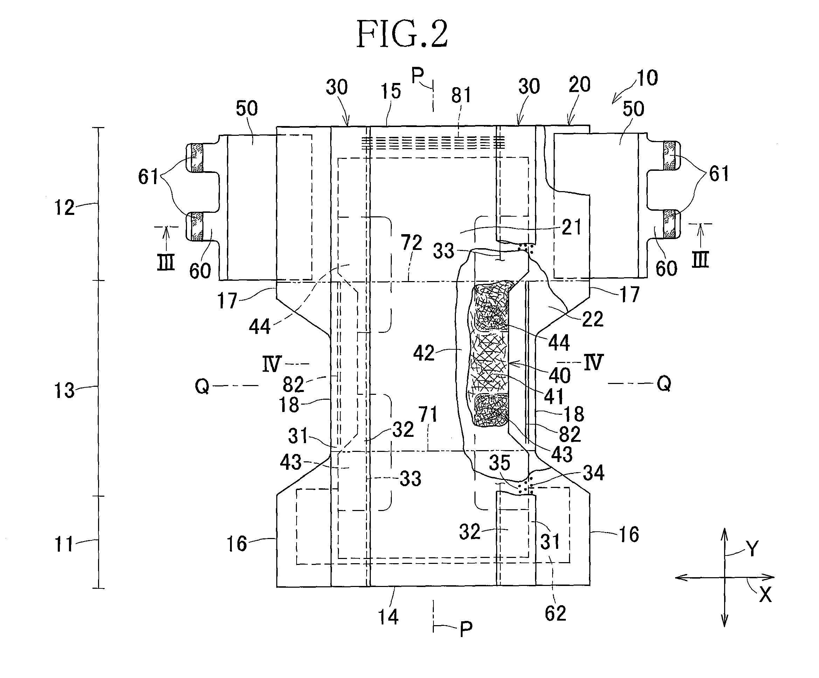

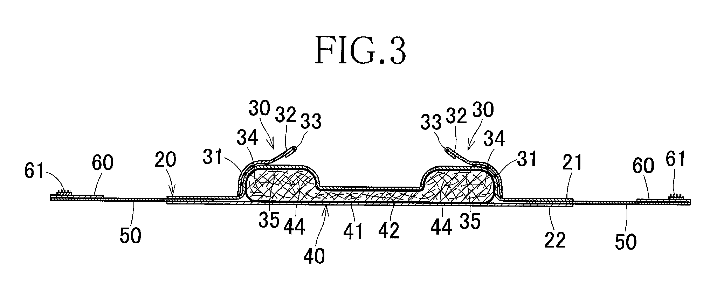

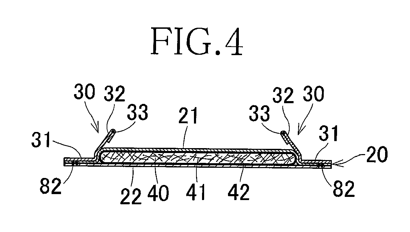

Figs. 1 through 4 illustrate an adult diaper 10 as an embodiment of the present invention. Fig. 1 is a perspective view of the diaper 10 having been sterically assembled, Fig. 2 is a developed plan view of the diaper 10 partially cutaway for convenience of illustration, Fig. 3 is a schematic sectional view taken along line III-III in Fig. 2 and Fig. 4 is a schematic sectional view taken along line IV-IV in Fig. 2. As will be apparent from Figs. 1 through 4, the diaper 10 has a longitudinal center line P-P bisecting a length dimension of the diaper 10 in the transverse direction X, a transverse center line Q-Q bisecting a length dimension of the diaper 10 in the longitudinal direction Y, a chassis 20 and a pair of leakage-barrier cuffs 30 lying on the side of the chassis 20 facing the wearer's body.

The diaper 10 comprises a front waist region 11, a rear waist region 12 and a crotch region 13 between these front and rear waist regions 11, 12 wherein these regions are continuous in the longitudinal direction Y. The front and rear waist regions 11, 12 respectively have front and rear ends 14, 15 which cooperate, in turn, with each other to define a waist-opening. The front waist region 11 has opposite side edges 16 extending in the longitudinal direction Y, the rear waist region 12 has opposite side edges 17 extending in the longitudinal direction Y and the crotch region 13 has opposite side edges 18 extending in the longitudinal direction Y. The opposite side edges 18 of the crotch region 13 define leg-openings.

The chassis 20 comprises an inner sheet 21 lying on the side facing the wearer's body and an outer sheet 22 lying on the side facing away from the wearer's body wherein a liquid-absorbent structure 40 is sandwiched between these inner and outer sheet 21, 22. The liquid-absorbent structure 40 comprises a liquid-absorbent core 41 and a wrapping sheet 42 used to wrap the liquid-absorbent core 41. The inner and outer sheet 21, 22 may be formed, for example, of a liquid-pervious fibrous nonwoven fabric, the liquid-absorbent core 41 may be formed, for example, of a mixture of fluff wood pulp and super-absorbent polymer particles and the wrapping sheet 42 may be formed, for example, of a liquid-dispersant tissue paper. It should be appreciated here that for these components, it is possible to use materials which are usually used in the related technical field.

Side sheets 50 are attached between the inner and outer sheet 21, 22 so that the respective side sheets 50 extend outward in the transverse direction X beyond the respective side edges 17. The sides of the respective side sheets 50 facing the wearer's body are provided with hook elements 61 as engagement members by the intermediary of sheet-like tab members 60. The side of the front waist region 11 facing away from the wearer's body is provided with loop elements 62 as engagement members adapted to be detachably engaged with the hook members 61. The loop elements 62 attached are attached to the front waist region 11 substantially over its entire range extending in the transverse direction X between the opposite side edges 16 so that the position at which the hook elements 61 are engaged with the loop elements 62 may be adjusted depending on the wearer's waist size.

Along the rear end 15, a plurality of waist elastics 81 is attached under tension and in a contractible manner in the transverse direction X between the inner and outer sheet 21, 22. Along the opposite side edges 18 which is located between the inner sheet and outer sheet 21,22 and at least within the crotch region 13, a plurality of leg elastics 82 is attached under tension and in a contractible manner in the longitudinal direction Y. These elastics 81, 82 are bonded to at least one of the inner and outer sheet 21, 22.

The liquid-absorbent structure 40 sandwiched between the inner and outer sheet 21, 22 extends across the crotch region 13 into the front and rear waist regions 11, 12 and, in the crotch region 13, has its opposite side edges cut away toward the longitudinal center line P-P. The liquid-absorbent structure 40 is formed with a highly absorbent region in which the liquid-absorbent structure 40 has a higher absorptive capacity than that in the remaining region. Specifically, the highly absorbent region comprises front regions 43 defined in the vicinity of the front end 14 and rear regions 44 defined in the vicinity of the rear end 15. These regions are partially formed by increasing a thickness of the liquid-absorbent core 41 in a thickness direction.

Each of the front and rear regions 43, 44 has a dimension in a range of 2 to 10 mm in the thickness direction. If the thickness is less than 2 mm, it will be difficult to differentiate these regions 43, 44 from the remaining region and if the thickness exceeds 10 mm, the liquid-absorbent structure 40 should become unacceptably stiff and/or the wearer might feel uncomfortable when wearing the diaper. A dimension difference between these front and rear regions 43, 4 and the remaining region is preferably about 0.5 mm or larger. Such dimensional difference advantageously assures the difference of the absorptive capacity between the regions 43, 44 and the remaining region and improves the bodily fluid absorption capacity in the highly absorbent regions.

In the present embodiment, the front and rear regions 43, 44 defined as the highly absorbent regions respectively have a thickness of about 9 mm and the bodily fluid absorption capacity is about 0.8g/cm². In contrast, the remaining region has a thickness of about 5 mm and the absorption capacity is about 0.4 g/cm². In this way, the highly absorbent region has an absorption capacity twofold higher than that of the remaining region.

A pair of the front region 43 and a pair of the rear regions 44 are spaced from each other in the longitudinal direction Y wherein the front regions 43 extend from the front waist region 11 toward the crotch region 13 and the rear regions 44 extend from the rear waist region 12 toward the crotch region 13. No highly absorbent region is formed between each of the front and rear regions 43, 44. The front regions 43 as well as the rear regions 44 are respectively spaced from each other in the transverse direction X and nearly symmetric about the longitudinal center line P-P. In other words, none of the highly absorbent regions is formed in the vicinity of the longitudinal center line P-P. If desired, a separately prepared liquid-absorbent pad (not shown) adapted to absorb bodily fluids such as urine may be attached to the side of the inner sheet 21 facing the wearer's body.

The paired leakage-barrier cuffs 30 are formed on the side of the inner sheet 21 facing the wearer's body. The paired leakage-barrier cuffs 30 are spaced from each other in the transverse direction X and extend substantially over the entire range from the front end 14 to the rear end 15. The leakage-barrier cuffs 30 may be formed, for example, of an air-permeable but liquid-impervious fibrous nonwoven fabric.
Such leakage-barrier cuffs 30 respectively include proximal side edges 31 lying outside as viewed in the transverse direction X and secured to the inner sheet 21, and free side edges 32 adapted to be spaced upward from the liquid-absorbent structure 40 by the intermediary of the inner sheet 21. The free side edges 32 are provided with cuff elastics 33 attached thereto so as to extend in the longitudinal direction Y.

The proximal side edges 31 of the respective leakage-barrier cuffs 30 are secured to the inner sheet 21 by suitable bonding means 34 which are provided substantially over the entire ranges extending from the front end 14 to the rear end 15. The proximal side edges 31 are defined by regions in which the leakage-barrier cuffs 30 are secured to the inner sheet 21 and partially overlap the front and rear regions 43, 44. It is preferable that boundaries of the proximal side edges 31 and free side edges 32 overlap the front and rear regions 43, 44. However, it should be appreciated that the proximal side edges 31 do not overlap the front and rear regions 43, 44 in the region defined between the front regions and the paired rear regions because the highly absorbent region is not present in this intermediate region.

When the diaper 10 is put on the wearer's body, the cuff elastics 33 contract and, under such contraction, the free side edges 32 of the leakage-barrier cuffs 30 are spaced upward from the inner sheet 21 so as to stand up toward the wearer's body (See Figs. 3 and 4). With the free side edges 32 spaced from the inner sheet 21 in this manner, the leakage-barrier cuffs 30 prevent leakage of bodily fluids in the transverse direction X. Even if discharged urine moves in the transverse direction X, the free side edges 32 of the leakage-barrier cuffs 30 spaced upward from the inner sheet 21 prevent such urine from leaking sideways in the transverse direction X.

Such urine blocked by the free side edges 32 in its further movement in the transverse direction X now flows down along the respective free side edges 32 to the liquid-absorbent structure 40. The free side edges 32 stand up toward the side facing the wearer's body on boundaries 35 defined between the free side edges 32 and the proximal side edges 31 secured to the inner sheet 21. In consequence, urine flows along the free side edges 32 toward the boundaries 35 on which the free side edges stand. The proximal side edges 31 overlap the front and rear regions 43, 44 of the liquid-absorbent structure 40, i.e., the boundaries 35 between the proximal side edges 31 and the free side edges 32 overlap the front and rear regions 43, 44 and therefore the urine flowing along the free side edges 32 reach the front and rear regions 43, 44 and is absorbed and contained by these regions 43, 44. In this way, the urine flowing along the free side edges 32 should not stay on the inner sides of the respective free side edges 32 and the wearer should not suffer from skin troubles caused thereby. Particularly because the front and rear regions 43, 44 are dimensioned to be larger than the remaining region, it is substantially assured that the urine flowing along the free side edges 32 is absorbed and contained by these front and rear regions 43, 44. In addition, these front and rear regions 43, 44 are dimensioned to be thicker than the remaining region and, in consequence, the urine reaches these regions in advance of reaching the remaining region. In this way, these front and rear regions 43, 44 contribute to quick absorption of urine.

The diaper 10 is folded up and packaged for shipment. For shipment, depending on the diaper's size, the diaper is folded in three in the longitudinal direction Y along fold lines 71, 72 extending in the transverse direction X with its side facing the wearer's body inside. After the diaper 10 is folded in three in this manner, fold traces may be left on the diaper along fold lines 71, 72 and portions defined by the fold lines may be spaced from the wearer's skin. If inner sheet 21 and liquid-absorbent structure 40 are spaced from the wearer' s body, gaps may be formed along the regions and urine moves through the gaps, and eventually urine leakage and/or skin troubles such as diaper rashes might be caused. However, these fold lines 71, 72 may be formed so as to overlap the front and rear regions 43, 44 of the liquid-absorbent structure 10 to prevent such gaps from being left since the bulky front and rear regions 43, 44 are reliably kept in close contact with the wearer's body. In this way, discharged urine can be fully absorbed and contained by these front and rear regions 43, 44 andurine leakage and/or skin troubles such as diaper rashes can be reliably prevented. In addition, the front and rear regions 43, 44 are locally made thicker and thereby these front and rear regions have a relatively high stiffness compared to the remaining region. Such higher stiffness prevents the fold traces from being left on the diaper along the fold lines 71, 72 and also prevents the diaper from being spaced from the wearer's body.

Meanwhile, if the fold traces are left in the diaper 10 along the fold lines 71, 72, a certain amount of urine tends to stay along these fold traces. However, by making the front and rear regions 43, 44 having a high absorbency overlap the fold lines 71, 72, the staying urine can be effectively absorbed. In addition, the fold lines 71, 72 may be formed in the region in which the proximal side edges 31 of the leakage-barrier cuffs 30 overlap the front and rear regions 43, 44 to assure that the urine staying in the vicinity of the fold lines 71, 72 can be prevented from leaking outward in the transverse direction X and the urine flowing along the free side edges 32 can be reliably absorbed and contained by the front and rear regions 43, 44. It should be noted that the fold lines 71, 72 are not limited to such arrangement as described above but the arrangement of the fold lines 71, 72 may be appropriately changed depending on the size of the diaper 10 and the manner of folding up of the diaper. Positional relationship between the fold lines 71, 72 and the front and rear regions 43, 44 and the other members may be also appropriately changed.

When the diaper 10 is folded in three as above mentioned, the fold lines 71, 72 are usually formed between the front and rear waist regions 11, 12 and the crotch region 13, respectively. Therefore, the front and rear regions 43, 44 may be continuously formed from the crotch region 13 toward the front and rear waist regions 11, 12 to match the fold lines 71, 72 to the front and rear regions 43, 44, respectively. When the diaper 10 is folded in two, the fold line will be single and usually formed in the vicinity of the transverse center line Q-Q. Therefore, the highly absorbent regions are preferably formed in the vicinity of the transverse center line Q-Q. In any way, the fold line or lines may be formed so as to correspond to the highly absorbent regions, and with such arrangement, the gaps between the wearer's body and the diaper can be prevented from being left, and even if a certain amount of urine stays, such urine can be reliably absorbed and contained. Such effect can be achieved also when the front and rear regions 43, 44 are continuous in the longitudinal direction instead of being discontinuous with each other.

The front and rear regions 43, 44 which are thicker than the remaining region are not formed in the vicinity of the longitudinal center line P-P and, in consequence, a depression is formed in the vicinity of the longitudinal center line P-P. When a separately prepared liquid-absorbent pad (not shown) is attached to the diaper 10, the depression defined in the vicinity of the longitudinal center line P-P facilitates such liquid-absorbent pad to attach to the diaper 10. By making the liquid-absorbent pad detachable from the diaper 10, the liquid-absorbent pad can be repetitively exchanged with fresh one and properly located merely by placing the pad in the depression.

The above-mentioned front and rear regions 43, 44 may be formed by forming the liquid-absorbent core 41 in the form of two layers and layering these in the thickness direction. The two layers of liquid-absorbent core 41 may be merely layered without using any particular tool or device, and consequently, no additional cost is required to form the front and rear regions 43, 44. In the remaining region, the quantity of the liquid-absorbent core 41 can be reduced and the corresponding cost can be reduced.

According to the present embodiment, the highly absorbent regions can be formed by layering the liquid-absorbent core 41 in the thickness direction.
However, it is essential the bodily fluid absorption capacity in these regions is higher than in the remaining region. Considering this, a basis mass of the liquid-absorbent core 41 may be increased to obtain the same effect. In this case, a basis mass of highly absorbent regions is preferably in a range of 100 to 550 g/m². If the basis mass of highly absorbent regions is less than 100 g/m², a difference in basis mass between the highly absorbent regions and the remaining region will be unacceptably small and if the basis mass exceeds 550 g/m², stiffness will be unacceptably high and the diaper might be uncomfortable to wear. The difference in basis mass between the highly absorbent regions and the remaining region is preferably 25 g/m²_{.}

When the highly absorbent regions are formed by increasing the basis mass, the liquid-absorbent core 41 can be increased without varying the thickness of the liquid-absorbent core 41 and thereby an uncomfortable feeling can be alleviated. When the basis mass is increased in this manner, stiffness of the highly absorbent regions can be increased and thereby formation of the fold traces can be restricted even if the wearing article is folded along the fold lines. By restricting formation of the fold traces, the gaps along which the liquid-absorbent structure 40 might be spaced from the wearer's body can be prevented from being left when the wearing article is put on the wearer's body. In addition, a density of the liquid-absorbent core in the highly absorbent regions is higher than that in the remaining region and a bodily fluid absorption rate is also higher in these highly absorbent regions than in the remaining region. Consequentially, even if bodily fluids is not completely absorbed and flows sideways in the transverse direction X, bodily fluids can be reliably absorbed by the highly absorbent regions.

According to the present invention, as the hook elements and the loop elements, "Velcro (trademark)" or "Magic Tape (trademark) " widely used in the technical field of the wearing article may be used. It should be noted that the engagement members are not limited to the hook elements and the loop elements but the other means may be used to join the front and rear waist regions.
The waist elastics 81 and the leg elastics 82 may be formed, for example, of a plurality of strand-like, string-like or tape-like material having rubber elasticity. Under contractile force of such elastic material, the waist-opening and the leg-openings can be kept in close contact with the wearer's body and thereby leakage of bodily fluids such as urine can be prevented. As these waist elastics and leg elastics 82, natural rubber or synthetic rubbers such as polyurethane each having the rubber elasticity may be used, and instead of such materials, generally used elastics such as a fibrous nonwoven fabric or a plastic sheet having the rubber elasticity may be also used. As the bonding means, widelyusedmeans such as adhesives or thermal or ultrasonic bonding techniques may be used.

While the present invention has been described above on the basis of an open-type diaper as a typical example, the present invention is applicable also to a pull-on pants-type diaper. While the case in which the separately prepared liquid-absorbent pad is attached to the side of the diaper facing the wearer's body has been described above, the present invention is not limited to this and applicable to the case in which the liquid-absorbent pad is not used.

### Reference Signs List

- 10: diaper
- 11: front waist region
- 12: rear waist region
- 13: crotch region
- 20: chassis
- 30: leakage-barrier cuffs
- 31: proximal side edge
- 32: free side edge
- 33: cuff elastics
- 40: liquid-absorbent structure
- 43: front region (highly absorbent region)
- 44: rear region (highly absorbent region)
- 71: fold line
- 72: fold line

## Claims

1. A wearing article comprising a chassis having a longitudinal direction, a transverse direction, a side facing the wearer's body, a side facing away from the wearer's body, a front waist region, a rear waist region and a crotch region extending between said front and rear waist regions wherein said front waist region, said crotch region and said rear waist region are continuous in said longitudinal direction, a liquid-absorbent structure located at least in said crotch region, and a pair of leakage-barrier cuffs lying on said side of said chassis facing the wearer's body and spaced from each other in said transverse direction, said wearing article being **characterized in that**:
said leakage-barrier cuffs respectively include proximal side edges extending in said longitudinal direction and bonded to said chassis, free side edges adapted to be spaced upward from said chassis and cuff elastics attached under tension and in a contractible manner in said longitudinal direction to said free side edges;
said liquid-absorbent structure includes highly absorbent regions having a higher bodily fluid absorption capacity than that in a remaining region; and
said proximal side edges are formed so as to overlap said highly absorbent regions.

2. The wearing article defined by Claim 1, wherein
said liquid-absorbent structure comprises a liquid-absorbent core and a wrapping sheet used to wrap said liquid-absorbent core; and
said highly absorbent regions are formed by increasing a thickness of said liquid-absorbent core so that said liquid-absorbent structure have a dimension in a thickness direction thereof thicker than said remaining region.

3. The wearing article defined by Claim 1, wherein
said liquid-absorbent structure comprises a liquid-absorbent core and a wrapping sheet used to wrap said liquid-absorbent core; and
said highly absorbent regions are formed by setting a higher basis mass of said liquid-absorbent core than a that of said remaining region.

4. The wearing article defined by any one of Claims 1 through 3, wherein
said wearing article having fold lines extending in said transverse direction and along which said wearing article is folded; and
said fold lines at least partially overlap said highly absorbent regions.

5. The wearing article defined by any one of Claims 1 through 4, wherein said highly absorbent regions comprise front and rear regions spaced one from another in said longitudinal direction and said front and rear regions are formed continuously from said crotch region toward said front and rear waist regions.

6. The wearing article defined by any one of Claims 1 through 5, wherein said highly absorbent regions are formed to be spaced one from another in said transverse direction.

7. The wearing article defined by any one of Claims 1 through 6, wherein said wearing article further includes a liquid-absorbent pad adapted to be placed on said side facing the wearer's body of said chassis between a pair of said leakage-barrier cuffs.
